# EUROPEAN PATENT APPLICATION

(11) **EP 4 810 482 A1**
(43) Date of publication of application: **23.09.2026**
(21) Application number: 24891749.4
(22) Date of filing: 12.11.2024
(51) Int. Cl.: C07D 207/267, B01D 3/14

(54) **PURIFICATION METHOD AND PURIFICATION APPARATUS FOR NMP**

(30) Priority: 13.11.2023 KR 20230156770
(71) Applicant: Duksan Co., Ltd., Anseong-si, Gyeonggi-do 17601 (KR); Hanmo Corporation, Seoul 08390 (KR)
(72) Inventor: LEE, Seungyong, Hongseong-gun, Chungcheongnam-do 32210 (KR); LEE, Wooyong, Yongin-si, Gyeonggi-do 16990 (KR); LEE, Heejae, Bucheon-si, Gyeonggi-do 14581 (KR); YOO, Insung, Seoul 08848 (KR)
(74) Representative: AWA Sweden AB
(86) International application number: PCT/KR2024/017843
(87) International publication number: WO 2025/105803

(57) **Abstract**

The present invention relates to a purification method and purification apparatus for NMP. Specifically, according to one embodiment of the present invention, the purification method for NMP purifies a gas mixture (A) containing NMP, separated out through a sludge separator, before performing an NMP purification step, and thus can improve processability, and the recovery rate and purity of NMP. In addition, a mixture containing sludge, separated out through a sludge separator, is injected into a filtrate recovery device and heat treated such that components having boiling points higher than that of NMP contained in the mixture containing sludge can be effectively removed, and a circulation step using an impurity tank is included to allow the components having boiling points higher than that of recirculated NMP to act as a transfer material of waste NMP, thereby facilitating use in an NMP purification process such that processability can be improved.

## Description

### Technical Field

The present invention relates to a process and an apparatus for purifying waste NMP.

### Background Art

In recent years, as technological development for portable devices, such as mobile phones and cameras, has been expedited, interest in secondary batteries as an energy source has increased. While demand for lithium secondary batteries, which have a high energy density and operating potential, a long cycle life, and a low self-discharge rate, is rapidly increasing, among such secondary batteries, research on their recycling is also ongoing.

N-methyl-2-pyrrolidone (NMP), an organic solvent, is widely used as a solvent in industries, such as petrochemicals, plastics, and pharmaceuticals, by virtue of its chemical stability, low volatility, and ability to dissolve various substances. It is also used in the process of manufacturing secondary batteries.

In particular, a lithium ion secondary battery is composed of a positive electrode manufactured by applying an electrode raw material comprising a lithium compound such as lithium cobaltate or lithium manganate, a binder such as polyvinylidene fluoride, and N-methyl-2-pyrrolidone (NMP) as a solvent to a substrate and firing the electrode raw material; and a negative electrode manufactured by applying an electrode raw material comprising a compound comprising carbon, titanium, and the like, a binder such as polyvinylidene fluoride, and water as a solvent to a substrate and firing the electrode raw material. As described above, since NMP is not only used as a main material for manufacturing positive electrodes in the electrode manufacturing process, but is also used in cleaning and drying steps, it is discharged in large quantities. Thus, research is ongoing on technologies to effectively recover it.

For example, Korean Patent No. 10-2050990 discloses a system for separating and recovering NMP from NMP gas in which NMP-containing gas that contains NMP is brought into gas-liquid contact with water that has absorbed NMP, thereby allowing NMP in the NMP-containing gas to be absorbed into the water.

### [Prior Art Document]

### [Patent Document]

(Patent Document 1) Korean Patent No. 10-2050990

### Detailed Description of the Invention

### Technical Problem

Accordingly, the present invention aims to provide a process and an apparatus for purifying NMP, which can enhance the recovery rate of NMP to be recovered from waste NMP and provide high-purity NMP.

### Solution to the Problem

A process for purifying NMP according to an embodiment of the present invention comprises: (1) feeding waste NMP from a raw material tank to a sludge separator; (2) discharging a gas mixture (A) containing NMP separated in the sludge separator through the top of the sludge separator; and discharging a mixture containing sludge through the bottom of the sludge separator; (3) feeding the discharged gas mixture (A) containing NMP to a first distillation column to perform first purification; (4) discharging an overhead product containing water separated by the first purification to the outside of the system through the top of the first distillation column; and discharging a first bottom product through the bottom of the first distillation column; (5) feeding the discharged first bottom product to a second distillation column to perform second purification; (6) discharging purified NMP separated by the second purification to the outside of the system through the top of the second distillation column; and discharging a second bottom product through the bottom of the second distillation column; (7) feeding the mixture containing sludge discharged in step (2) to a filtrate recovery device to perform thermal treatment thereof; (8) discharging a third bottom product separated by the thermal treatment to the outside of the system through the bottom of the filtrate recovery device; and discharging a gas mixture (B) containing NMP through the top of the filtrate recovery device; (9) feeding the second bottom product discharged in step (6) and the gas mixture (B) containing NMP discharged in step (8) to an impurity tank and mixing them; and (10) circulating the mixture from the impurity tank to the raw material tank.

An apparatus for purifying NMP according to another embodiment of the present invention comprises: a raw material tank for supplying waste NMP; a sludge separator having a sludge filter and a sludge separator plate therein for discharging a gas mixture (A) containing NMP separated from the supplied waste NMP through its top and a mixture containing sludge through its bottom; a first distillation column for performing first purification of the gas mixture (A) containing NMP, discharging an overhead product containing water separated by the first purification to the outside of the system through its top, and discharging a first bottom product through its bottom; a second distillation column for performing second purification of the first bottom product through distillation, discharging purified NMP separated by the second purification to the outside of the system through its top, and discharging a second bottom product through its bottom; a filtrate recovery device for performing thermal treatment of the mixture containing sludge discharged from the sludge separator, discharging a third bottom product separated by the thermal treatment to the outside of the system through its bottom, and discharging a gas mixture (B) containing NMP through its top; and an impurity tank for mixing the second bottom product discharged from the second distillation column and the gas mixture (B) containing NMP discharged from the filtrate recovery device, and feeding the mixture into the raw material tank.

### Advantageous Effects of the Invention

The process for purifying NMP according to an embodiment of the present invention can enhance the recovery rate of NMP to be recovered from waste NMP, thereby providing high-purity NMP.

Specifically, waste NMP recovered once it has been used as a raw material for lithium secondary batteries may contain a large amount of sludge. Waste NMP containing such a large amount of sludge is not readily transported in the process of recovering NMP, so that it may clog the transport pipe, and a separate measure may be required for transporting it.

In the process for purifying NMP according to an embodiment of the present invention, the gas mixture (A) containing NMP separated in the sludge separator is purified before the NMP purification step is carried out, whereby the processability, as well as the recovery rate and purity of NMP, can be enhanced. In particular, as the sludge separator comprises a sludge filter and a sludge separator plate, a large amount of the sludge contained in the waste NMP can be effectively separated without special control of process conditions.

In addition, as the mixture containing the sludge separated in the sludge separator is fed to the filtrate recovery device and thermally treated therein, components having a boiling point higher than that of NMP contained in the mixture, containing the sludge, can be effectively removed.

In addition, the second bottom product discharged in the second purification step and the gas mixture (B) containing NMP discharged from the filtrate recovery device are fed to the impurity tank and then mixed, and the mixture is circulated to the raw material tank. As a result, not only can the recovery rate and purity of NMP be enhanced, but also the components with a higher boiling point than that of the circulated NMP are used as a transport material for waste NMP, whereby their use in the NMP purification process is facilitated, resulting in enhanced processability.

In addition, when the mixture of the impurity tank is thermally treated and fed to the second distillation column, the recovery rate of NMP can be further enhanced.

### Brief Description of the Drawing

Fig. 1 schematically illustrates a flow chart of an NMP purification process according to an embodiment of the present invention.
Fig. 2 illustrates an example of an NMP purification apparatus according to another embodiment of the present invention.

### Best Mode for Carrying out the Invention

Hereinafter, the present invention will be described in detail. The present invention is not limited to the disclosures given below, but it may be modified into various forms as long as the gist of the invention is not changed.

Throughout the present specification, when a part is referred to as "comprising" an element, it is understood that other elements may be comprised, rather than other elements are excluded, unless specifically stated otherwise.

All numbers and expressions related to the quantities of components, reaction conditions, and the like used herein are to be understood as being modified by the term "about," unless otherwise indicated.

In the present specification, in the case where an element is mentioned to be formed "on" or "under" another element, it means not only that one element is directly formed "on" or "under" another element, but also that one element is indirectly formed on or under another element with other element(s) interposed between them.

Throughout the present specification, the terms first, second, and the like are used to describe various components. But the components should not be limited by the terms. The terms are used only for the purpose of distinguishing one component from another.

### NMP purification process

The process for purifying NMP according to an embodiment of the present invention comprises: (1) feeding waste NMP from a raw material tank to a sludge separator; (2) discharging a gas mixture (A) containing NMP separated in the sludge separator through the top of the sludge separator; and discharging a mixture containing sludge through the bottom of the sludge separator; (3) feeding the discharged gas mixture (A) containing NMP to a first distillation column to perform first purification; (4) discharging an overhead product containing water separated by the first purification to the outside of the system through the top of the first distillation column; and discharging a first bottom product through the bottom of the first distillation column; (5) feeding the discharged first bottom product to a second distillation column to perform second purification; (6) discharging purified NMP separated by the second purification to the outside of the system through the top of the second distillation column; and discharging a second bottom product through the bottom of the second distillation column; (7) feeding the mixture containing sludge discharged in step (2) to a filtrate recovery device to perform thermal treatment thereof; (8) discharging a third bottom product separated by the thermal treatment to the outside of the system through the bottom of the filtrate recovery device; and discharging a gas mixture (B) containing NMP through the top of the filtrate recovery device; (9) feeding the second bottom product discharged in step (6) and the gas mixture (B) containing NMP discharged in step (8) to an impurity tank and mixing them; and (10) circulating the mixture from the impurity tank to the raw material tank.

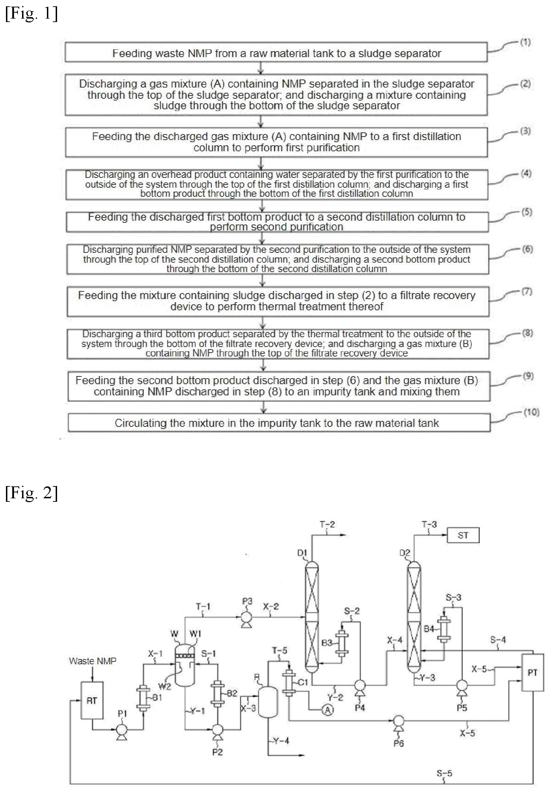

Fig. 1 schematically illustrates an NMP purification process according to an embodiment of the present invention. Fig. 2 illustrates an example of an NMP purification apparatus according to another embodiment of the present invention. Specifically, Fig. 2 illustrates an NMP purification apparatus composed of a raw material tank (RT), a sludge separator (W), a first distillation column (D1), a second distillation column (D2), a filtrate recovery device (R), and an impurity tank (PT).

Hereinafter, the process for purifying NMP according to an embodiment of the present invention will be described with reference to Figs. 1 and 2.

### Step (1) of feeding waste NMP from a raw material tank to a sludge separator

The process for purifying NMP according to an embodiment of the present invention comprises a step of feeding waste NMP from a raw material tank to a sludge separator.

The waste NMP may be recovered once it has been used in the process of manufacturing a lithium secondary battery, but it is not limited thereto. For example, the waste NMP may be recovered once it has been used as a raw material for lithium secondary batteries and may contain a large amount of sludge.

The waste NMP may contain NMP in an amount of 70% by weight or more. For example, the content of NMP in the waste NMP may be 72% by weight or more, 75% by weight or more, 78% by weight or more, 80% by weight or more, 85% by weight or more, 90% by weight or more, or 95% by weight or more, based on the total weight of the waste NMP.

In addition, the waste NMP may contain water. For example, the content of water in the waste NMP may be 30% by weight or less, 25% by weight or less, 20% by weight or less, or 15% by weight or less, based on the total weight of the waste NMP.

The sludge separator may comprise a sludge filter and a sludge separator plate. Specifically, the sludge filter may be in the form of a mesh net located in the upper part inside the sludge separator. In addition, the sludge separator plate may be provided at a position lower than the sludge filter, and it may be provided at a position where the waste NMP is fed into the sludge separator. The sludge separator plate serves to prevent the flow of waste NMP fed into the sludge separator from coming into direct contact with the sludge filter, and it may have a shape of " 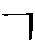 ," but it is not limited thereto. The sludge separator plate blocks the flow of waste NMP fed into the sludge separator to primarily filter sludge, thereby not only removing sludge with high efficiency but also extending the lifespan of the sludge filter.

While the waste NMP passes through the sludge separator plate and the sludge filter, a gas mixture (A) containing the NMP can be discharged through the top of the sludge separator, and a large amount of sludge contained in the waste NMP can be effectively separated through the sludge separator plate and the sludge filter.

As the process for purifying NMP according to an embodiment of the present invention uses the sludge separator having the technical features described above, it can simply separate a large amount of sludge contained in waste NMP without special control of process conditions.

In addition, the process may further comprise, before feeding the waste NMP to the sludge separator in step (1), a step (1-1) of preheating the waste NMP to a temperature of 60°C to 100°C. For example, step (1-1) may be a step of preheating the waste NMP to a temperature of 62°C to 100°C or 65°C to 95°C. The preheated waste NMP may be fed to the sludge separator.

### Step (2) of discharging a gas mixture (A) containing NMP separated in the sludge separator through the top of the sludge separator; and discharging a mixture containing sludge through the bottom of the sludge separator

The process for purifying NMP according to an embodiment of the present invention comprises a step of discharging a gas mixture (A) containing NMP separated in the sludge separator through the top of the sludge separator; and discharging a mixture containing sludge through the bottom of the sludge separator.

Specifically, in step (2), a gas mixture (A) containing NMP separated using the sludge separator and a mixture containing sludge are effectively separated, whereby a large amount of the sludge contained in the waste NMP is removed to enhance the processability of recovering NMP and the recovery rate and purity of NMP.

### Step (3) of feeding the discharged gas mixture (A) containing NMP to a first distillation column to perform first purification

The process for purifying NMP according to an embodiment of the present invention comprises a step of feeding the discharged gas mixture (A) containing NMP to a first distillation column to perform first purification.

In step (3), the discharged gas mixture (A) containing NMP may be fed to the middle or upper part of the first distillation column. Specifically, the gas mixture (A) containing NMP may preferably be fed to the middle position, or an upper position, of the first distillation column in order to perform the first purification more efficiently, but it is not limited thereto.

The first purification in step (3) may be performed at a temperature of 50°C to 100°C and a pressure of 100 Torr to 130 Torr. For example, the first purification may be performed at a temperature of 52°C to 85°C or 55°C to 75°C and a pressure of 105 Torr to 130 Torr or 110 Torr to 125 Torr. As the temperature and pressure conditions of the first purification satisfy the above ranges, water contained in the gas mixture (A) containing NMP can be effectively removed.

### Step (4) of discharging an overhead product containing water separated by the first purification to the outside of the system through the top of the first distillation column; and discharging a first bottom product through the bottom of the first distillation column

The process for purifying NMP according to an embodiment of the present invention comprises a step of discharging an overhead product containing water separated by the first purification to the outside of the system through the top of the first distillation column; and discharging a first bottom product through the bottom of the first distillation column.

Specifically, in step (4), the overhead product containing water is separated from the gas mixture (A) containing NMP in the first purification of step (3) and discharged to the outside of the system, which reduces the content of water, thereby enhancing the purity of NMP.

The content of water in the first bottom product may be 0.01% or less. For example, the content of water in the first bottom product may be 0.008% or less, 0.006% or less, or 0.005% or less.

In addition, the first bottom product may contain NMP in an amount of 99.9% by weight or more. For example, the content of NMP in the first bottom product may be 99.92% by weight or more, 99.94% by weight or more, or 99.95% by weight or more, based on the total weight of the first bottom product. As most of the water in the waste NMP is effectively removed in the first purification described above, the purity of NMP in the first bottom product can be further enhanced.

### Step (5) of feeding the discharged first bottom product to a second distillation column to perform second purification

The process for purifying NMP according to an embodiment of the present invention comprises a step of feeding the discharged first bottom product to a second distillation column to perform second purification.

The purity of NMP can be enhanced through the second purification. Specifically, the waste NMP may contain a component with a boiling point higher than that of NMP, along with NMP. As the second purification is performed in step (5) on the first bottom product, from which water has been removed in the first purification, the component having a boiling point higher than that of NMP can be effectively removed, thereby further enhancing the purity of NMP.

In step (5), the first bottom product may be fed to the middle or lower part of the second distillation column. Specifically, the first bottom product may preferably be fed to the middle position, or a lower position, of the second distillation column in order to perform the second purification more efficiently, but it is not limited thereto.

The second purification may be performed at a temperature of 120°C to 150°C and a pressure of 50 Torr to 90 Torr. For example, the second purification may be performed at a temperature of 122°C to 148°C or 126°C to 145°C and a pressure of 55 Torr to 85 Torr or 65 Torr to 80 Torr. As the temperature and pressure conditions of the second purification satisfy the above ranges, the component having a boiling point higher than that of NMP and contained in the first bottom product can be effectively removed.

### Step (6) of discharging purified NMP separated by the second purification to the outside of the system through the top of the second distillation column; and discharging a second bottom product through the bottom of the second distillation column

The process for purifying NMP according to an embodiment of the present invention comprises a step of discharging purified NMP separated by the second purification to the outside of the system through the top of the second distillation column; and discharging a second bottom product through the bottom of the second distillation column.

Specifically, in step (6), as a second bottom product containing the component with a boiling point higher than that of NMP is separated from the first bottom product by the second purification in step (5) and discharged, purified NMP can be obtained.

The second bottom product may contain the component having a boiling point higher than that of NMP in an amount of 1.0% by weight or less. For example, the content of the component having a boiling point higher than that of NMP in the second bottom product may be 0.8% by weight or less, 0.6% by weight or less, or 0.4% by weight or less, based on the total weight of the second bottom product.

The second bottom product may contain NMP in an amount of 99% by weight or more. For example, the content of NMP in the second bottom product may be 99.2% by weight or more, 99.4% by weight or more, 99.5% by weight or more, or 99.6% by weight or more, based on the total weight of the second bottom product.

The purified NMP may have a purity of 99.9% or higher. For example, the purity of the purified NMP may be 99.92% or more, 99.94% or more, or 99.95% or more.

### Step (7) of feeding the mixture containing sludge discharged in step (2) to a filtrate recovery device to perform thermal treatment thereof

The process for purifying NMP according to an embodiment of the present invention comprises a step of feeding the mixture containing sludge discharged in step (2) to a filtrate recovery device to perform thermal treatment thereof.

Specifically, the mixture containing sludge separated in the sludge separator may also contain NMP. Thus, as the mixture containing sludge is fed to the filtrate recovery device and thermally treated therein, NMP contained in the mixture containing the sludge can be separated in a gaseous form and recirculated.

In step (7), the thermal treatment may be performed at a temperature of 110°C to 140°C and a pressure of 60 Torr to 100 Torr under stirring at 20 rpm to 80 rpm. For example, the thermal treatment may be performed at a temperature of 115°C to 140°C, 125°C to 138°C, or 128°C to 135°C, and a pressure of 65 Torr to 95 Torr or 75 Torr to 85 Torr, under stirring at 25 rpm to 70 rpm or 30 rpm to 60 rpm. As the thermal treatment conditions satisfy the above ranges, the recovery rate of NMP can be further enhanced.

In addition, the pressure of the filtrate recovery device may be controlled by a vacuum pump connected to the filtrate recovery device, more specifically, a vacuum pump connected to a cooler connected to the filtrate recovery device. It is important to control the pressure of the filtrate recovery device in order to produce the effect desired in the present invention; therefore, the pressure of the filtrate recovery device may be controlled by a vacuum pump.

### Step (8) of discharging a third bottom product separated by the thermal treatment to the outside of the system through the bottom of the filtrate recovery device; and discharging a gas mixture (B) containing NMP through the top of the filtrate recovery device

The process for purifying NMP according to an embodiment of the present invention comprises a step of discharging a third bottom product separated by the thermal treatment to the outside of the system through the bottom of the filtrate recovery device; and discharging a gas mixture (B) containing NMP through the top of the filtrate recovery device.

Specifically, in step (8), a third bottom product and a gas mixture (B) containing NMP can be separated by thermal treatment in step (7), and the gas mixture (B) containing NMP can be circulated to be utilized, whereby the processability and the recovery rate of NMP can be enhanced.

The gas mixture (B) containing NMP may contain the component having a boiling point higher than that of NMP in an amount of 1.5% by weight or less. For example, the content of the component having a boiling point higher than that of NMP in the gas mixture (B) containing NMP may be 1.2% by weight or less, 1.0% by weight or less, 0.8% by weight or less, 0.6% by weight or less, or 0.5% by weight or less, based on the total weight of the gas mixture (B) containing NMP.

### Step (9) of feeding the second bottom product discharged in step (6) and the gas mixture (B) containing NMP discharged in step (8) to an impurity tank and mixing them

The process for purifying NMP according to an embodiment of the present invention comprises a step of feeding the second bottom product discharged in step (6) and the gas mixture (B) containing NMP discharged in step (8) to an impurity tank and mixing them.

The mixture in the impurity tank may contain the component having a boiling point higher than that of NMP in an amount of 1.5% by weight or less. For example, the content of the component having a boiling point higher than that of NMP in the mixture in the impurity tank may be 1.0% by weight or less, 0.8% by weight or less, 0.6% by weight or less, or 0.5% by weight or less, based on the total weight of the mixture in the impurity tank.

According to another embodiment of the present invention, the process may further comprise a step (9-1) of cooling the gas mixture (B) containing NMP discharged in step (9) to 45°C or lower before feeding it to the impurity tank.

The gas mixture (B) containing NMP discharged in step (9) may be fed to the impurity tank without separate treatment and then circulated. However, in order to facilitate mixing with the second bottom product discharged in step (6), a step (9-1) of cooling it to 42°C or lower, 40°C or lower, or 35°C or lower, using a cooler, may be further performed before it is fed to the impurity tank.

### Step (10) of circulating the mixture from the impurity tank to the raw material tank

The process for purifying NMP according to an embodiment of the present invention comprises a step of circulating the mixture from the impurity tank to the raw material tank.

Specifically, as the mixture in the impurity tank is circulated as it is fed to the raw material tank, the recovery rate and purity of NMP can be enhanced. In particular, as the component with a boiling point higher than that of NMP is used as a transport material for waste NMP, its use in the NMP purification process is facilitated, resulting in enhanced processability.

According to another embodiment of the present invention, the process may further comprise a step (11) of performing thermal treatment of the mixture in the impurity tank to feed it to the second distillation column. Specifically, the mixture in the impurity tank may be fed to the raw material tank to be circulated, and it may be thermally treated and then fed to the second distillation column to be circulated, whereby the recovery rate of NMP can be further enhanced.

In step (11), the thermal treatment may be performed at 100°C to 130°C. For example, the thermal treatment in step (11) may be performed at a temperature of 105°C to 130°C or 115°C to 130°C.

In addition, the mixture of the impurity tank thermally treated in step (11) may be fed to the middle or lower part of the second distillation column. Specifically, the gas mixture may preferably be fed to the middle position, or a lower position, of the second distillation column in order to perform more effective purification, but it is not limited thereto.

More specifically, the position at which the mixture of the impurity tank thermally treated in step (11) is fed to the second distillation column may be the same as, or lower than, the position at which the first bottom product is fed to the second distillation column in step (5). As the position at which the mixture of the impurity tank thermally treated in step (11) is fed to the second distillation column is the same as, or lower than, the position at which the first bottom product is fed to the second distillation column in step (5), NMP can be purified more effectively. In particular, since high-purity NMP, as a final product, is discharged to the outside of the system through the top of the second distillation column, the mixture of the first bottom product and the thermally treated mixture of the impurity tank must be fed to the middle position, or a lower position, of the second distillation column to increase the efficiency of NMP purification. As the feeding position of the thermally treated mixture of the impurity tank having a low content of NMP is set to be relatively lower than the feeding position of the first bottom product, more efficient purification can be carried out.

### NMP purification apparatus

The apparatus for purifying NMP according to another embodiment of the present invention comprises: a raw material tank for supplying waste NMP; a sludge separator having a sludge filter and a sludge separator plate therein for discharging a gas mixture (A) containing NMP separated from the supplied waste NMP through its top and a mixture containing sludge through its bottom; a first distillation column for performing first purification of the gas mixture (A) containing NMP, discharging an overhead product containing water separated by the first purification to the outside of the system through its top, and discharging a first bottom product through its bottom; a second distillation column for performing second purification of the first bottom product through distillation, discharging purified NMP separated by the second purification to the outside of the system through its top, and discharging a second bottom product through its bottom; a filtrate recovery device for performing thermal treatment of the mixture containing sludge discharged from the sludge separator, discharging a third bottom product separated by the thermal treatment to the outside of the system through its bottom, and discharging a gas mixture (B) containing NMP through its top; and an impurity tank for mixing the second bottom product discharged from the second distillation column and the gas mixture (B) containing NMP discharged from the filtrate recovery device, and feeding the mixture into the raw material tank.

Fig. 2 illustrates an example of an NMP purification apparatus according to another embodiment of the present invention. Specifically, Fig. 2 illustrates an NMP purification apparatus composed of a raw material tank (RT), a sludge separator (W), a first distillation column (D1), a second distillation column (D2), a filtrate recovery device (R), and an impurity tank (PT).

First, waste NMP is supplied to a sludge separator (W) through a raw material tank (RT) (X-1). The supply may be carried out through a first pump (P1). In such an event, the waste NMP may be preheated through a preheater (B1).

The sludge separator (W) may comprise a sludge filter (W1) provided in the upper part of the interior and a sludge separator plate (W2) provided on the upper side of the interior at a lower position than the sludge filter.

A gas mixture (A) containing NMP, separated from the waste NMP or preheated waste NMP by the sludge filter (W1) and the sludge separator plate (W2) in the sludge separator (W), is discharged through the top of the sludge separator (T-1) and fed to a first distillation column (D1) through a third pump (P3) (X-2), and a mixture containing sludge is discharged through the bottom of the sludge separator (Y-1). The discharged mixture containing sludge may be transferred to a filtrate recovery device (R) through a second pump (P2) (X-3) and then circulated to the sludge separator (W) again (S-1). In such an event, the mixture containing sludge may be preheated through a heater (B2) for the sludge separator before it is fed back to the sludge separator (W).

The first distillation column (D1) performs first purification by distilling the fed gas mixture (A) containing NMP. An overhead product containing water separated by the first purification is discharged to the outside of the system through the top of the first distillation column (D1) (T-2), and a first bottom product is discharged through the bottom of the first distillation column (D1) (Y-2). The discharged first bottom product may be transferred to a second distillation column (D2) through a fourth pump (P4) (X-3) and may be circulated back to the first distillation column (D1) (S-2). In such an event, the first bottom product may be preheated through a first reboiler (B3) before it is fed back to the first distillation column (D1).

The second distillation column (D2) performs second purification by distilling the fed first bottom product. Purified NMP separated by the second purification is discharged to the outside of the system through the top of the second distillation column (D2) (T-3), and a second bottom product is discharged through the bottom of the second distillation column (D2) (Y-3). The purified NMP discharged from the system may be stored in a storage tank (ST). In addition, the discharged second bottom product may be transferred to an impurity tank (PT) through a fifth pump (P5) (X-4) and circulated back to the second distillation column (D2) (S-3). In such an event, the second bottom product may be preheated through a second reboiler (B4) before it is fed back to the second distillation column (D2).

The filtrate recovery device (R) performs thermal treatment of the fed mixture containing sludge. In such an event, the pressure may be controlled through a vacuum pump (A). A third bottom product separated by the thermal treatment is discharged to the outside of the system through the bottom of the filtrate recovery device (Y-4), and a gas mixture (B) containing NMP is discharged through the top of the filtrate recovery device (T-5) and fed to the impurity tank (PT) through a sixth pump (P6) (X-5). In such an event, the discharged gas mixture (B) containing NMP may be cooled through a cooler (C1) and then fed to the impurity tank (PT).

In the impurity tank (PT), the second bottom product discharged from the second distillation column (D2) and the gas mixture (B) containing NMP discharged from the filtrate recovery device (R) are mixed by stirring, and the mixture is then circulated to the raw material tank (RT) (S-5). In such an event, a thermal treatment device may be provided to perform thermal treatment of the mixture of the impurity tank and feed it to the second distillation column (D2) (S-4).

### [Explanation of Reference Numerals]

D1, D2: first and second distillation columns
P1 to P6: first to sixth pumps
B1: preheater
B2: heater for the sludge separator
B3: first reboiler
B4: second reboiler
W: sludge separator
W1: sludge filter
W2: sludge separator plate
R: filtrate recovery device
RT: raw material tank
PT: impurity tank
ST: storage tank
T-1 to T-5, Y-1 to Y-4: discharge steps
S-1 to S-5: circulation steps
X-1 to X-5: feeding steps
A: vacuum pump

## Claims

1. A process for purifying NMP, which comprises:
(1) feeding waste NMP from a raw material tank to a sludge separator;
(2) discharging a gas mixture (A) containing NMP separated in the sludge separator through the top of the sludge separator; and discharging a mixture containing sludge through the bottom of the sludge separator;
(3) feeding the discharged gas mixture (A) containing NMP to a first distillation column to perform first purification;
(4) discharging an overhead product containing water separated by the first purification to the outside of the system through the top of the first distillation column; and discharging a first bottom product through the bottom of the first distillation column;
(5) feeding the discharged first bottom product to a second distillation column to perform second purification;
(6) discharging purified NMP separated by the second purification to the outside of the system through the top of the second distillation column; and discharging a second bottom product through the bottom of the second distillation column;
(7) feeding the mixture containing sludge discharged in step (2) to a filtrate recovery device to perform thermal treatment thereof;
(8) discharging a third bottom product separated by the thermal treatment to the outside of the system through the bottom of the filtrate recovery device; and discharging a gas mixture (B) containing NMP through the top of the filtrate recovery device;
(9) feeding the second bottom product discharged in step (6) and the gas mixture (B) containing NMP discharged in step (8) to an impurity tank and mixing them; and
(10) circulating the mixture from the impurity tank to the raw material tank.

2. The process for purifying NMP according to claim 1, wherein the sludge separator comprises a sludge filter and a sludge separator plate.

3. The process for purifying NMP according to claim 1, which further comprises, before feeding the waste NMP to the sludge separator in step (1), a step (1-1) of preheating the waste NMP to a temperature of 60°C to 100°C.

4. The process for purifying NMP according to claim 1, wherein the mixture in the impurity tank contains a component having a boiling point higher than that of NMP in an amount of 1.5% by weight or less.

5. The process for purifying NMP according to claim 1, which further comprises a step (11) of performing thermal treatment of the mixture of the impurity tank to feed it to the second distillation column.

6. The process for purifying NMP according to claim 5, wherein, in step (11), the thermal treatment is performed at 100°C to 130°C.

7. The process for purifying NMP according to claim 5, wherein the position at which the mixture of the impurity tank thermally treated in step (11) is fed to the second distillation column is the same as, or lower than, the position at which the first bottom product is fed to the second distillation column in step (5).

8. The process for purifying NMP according to claim 1, wherein the pressure of the filtrate recovery device is controlled by a vacuum pump connected to the filtrate recovery device.

9. The process for purifying NMP according to claim 1, which further comprises a step (9-1) of cooling the gas mixture (B) containing NMP discharged in step (9) to 45°C or lower before feeding it to the impurity tank.

10. The process for purifying NMP according to claim 1, wherein the gas mixture (B) containing NMP contains a component having a boiling point higher than that of NMP in an amount of 1.5% by weight or less.

11. The process for purifying NMP according to claim 1, wherein the waste NMP contains NMP in an amount of 70% by weight or more.

12. The process for purifying NMP according to claim 1, wherein, in step (3), the discharged gas mixture (A) containing NMP is fed to the middle or upper part of the first distillation column.

13. The process for purifying NMP according to claim 1, wherein the first bottom product contains NMP in an amount of 99.9% by weight or more.

14. The process for purifying NMP according to claim 1, wherein the content of water in the first bottom product is 0.01% or less.

15. The process for purifying NMP according to claim 1, wherein, in step (5), the first bottom product is fed to the middle or lower part of the second distillation column.

16. The process for purifying NMP according to claim 1, wherein the second bottom product contains NMP in an amount of 99% by weight or more.

17. The process for purifying NMP according to claim 1, wherein the first purification in step (3) is performed at a temperature of 50°C to 100°C and a pressure of 100 Torr to 130 Torr, and the second purification in step (5) is performed at a temperature of 120°C to 150°C and a pressure of 50 Torr to 90 Torr.

18. The process for purifying NMP according to claim 1, wherein the purified NMP has a purity of 99.9% or higher.

19. An apparatus for purifying NMP, which comprises:
a raw material tank for supplying waste NMP;
a sludge separator having a sludge filter and a sludge separator plate therein for discharging a gas mixture (A) containing NMP separated from the supplied waste NMP through its top and a mixture containing sludge through its bottom;
a first distillation column for performing first purification of the gas mixture (A) containing NMP, discharging an overhead product containing water separated by the first purification to the outside of the system through its top, and discharging a first bottom product through its bottom;
a second distillation column for performing second purification of the first bottom product through distillation, discharging purified NMP separated by the second purification to the outside of the system through its top, and discharging a second bottom product through its bottom;
a filtrate recovery device for performing thermal treatment of the mixture containing sludge discharged from the sludge separator, discharging a third bottom product separated by the thermal treatment to the outside of the system through its bottom, and discharging a gas mixture (B) containing NMP through its top; and
an impurity tank for mixing the second bottom product discharged from the second distillation column and the gas mixture (B) containing NMP discharged from the filtrate recovery device, and feeding the mixture into the raw material tank.

20. The apparatus for purifying NMP according to claim 19, which further comprises a vacuum pump for controlling the pressure of the filtrate recovery device.

21. The apparatus for purifying NMP according to claim 19, which further comprises a thermal treatment device for performing thermal treatment of the mixture of the impurity tank and feeding it to the second distillation column.
